# EUROPEAN PATENT APPLICATION

(11) **EP 1 854 466 A1**
(43) Date of publication of application: **14.11.2007**
(21) Application number: 07112582.7
(22) Date of filing: 11.12.2003
(51) Int. Cl.: A61K 31/59, A61K 31/593, A61K 31/575, A61P 17/06, A61P 17/00

(54) **Pharmaceutical composition comprising a combination of calcitriol and clobetasol propionate**

(30) Priority: 17.12.2002 FR 0216016; 31.12.2002 US 437057 P
(62) Divisional of application: 03789441.7
(71) Applicant: Galderma Research & Development, S.N.C., 06410 Biot (FR)
(72) Inventor: Jomard, André, 06460, Saint Vallier de Thiey (FR); Roye, Olivier, 06370, Mouans Sartoux (FR)
(74) Representative: Andral, Christophe André Louis

(57) **Abstract**

The present invention relates to pharmaceutical compositions in the form of a gel, a cream, a lotion, a solution or an ointment comprising, in a pharmaceutically acceptable medium, at least calcitriol and clobetasol propionate, and to the use of those compositions for the preparation of a medicinal product for treating dermatological complaints such as psoriasis, atopic dermatitis, contact dermatitis and seborrhoeic dermatitis.

## Description

The present invention relates to pharmaceutical compositions in the form of a gel, a cream, a lotion, a solution or an ointment comprising, in a pharmaceutically acceptable medium, at least calcitriol and clobetasol propionate, and to the use of those compositions for the preparation of a medicinal product for treating dermatological complaints such as psoriasis, atopic dermatitis, contact dermatitis and seborrhoeic dermatitis.

More specifically, the invention relates to pharmaceutical compositions comprising at least calcitriol and clobetasol propionate, in a given ratio such that a synergistic effect between the two active principles is observed in the treatment of dermatological complaints such as psoriasis, atopic dermatitis, contact dermatitis and seborrhoeic dermatitis.

The combination of active principles is not used conventionally in the treatment of dermatological complaints. This is generally due to the difficulty encountered by a person skilled in the art during the combination of two active principles as regards the chemical stability and the interactions that the medicinal products may present when they are present in the same formulation.

Calcitriol is a vitamin D analogue used to regulate the level of calcium in the body. Its use in the treatment of dermatological diseases has especially been described in patent US 4 610 978 for the treatment of psoriasis. The said patent suggests compositions comprising calcitriol that may also contain an amount of an antiinflammatory agent such as clobetasol propionate; however, no concrete embodiment for combining calcitriol and a corticosteroid is described or tested in terms of efficacy. Consequently, it was not obvious to a person skilled in the art to anticipate that the combination of calcitriol with clobetasol propionate would have any synergistic effect.

WO 00/64450 mentions the use of a pharmaceutical composition containing a vitamin D analogue and clobetasol propionate. All the examples of compositions in the said patent application combine calcipotriol and betamethasone. The comparison of the measurements of the efficacy, on patients suffering from psoriasis of a composition comprising calcipotriol alone, betamethasone alone or a combination of the two active agents shows that the effect obtained by the combination corresponds to an additive effect. Thus, with regard to the said document, a person skilled in the art could not in any way have imagined that the combination of a vitamin D analogue with a corticosteroid could have a synergistic effect.

The Applicant has discovered, surprisingly, that the combination of calcitriol with clobetasol propionate affords a synergistic effect in the treatment of certain dermatological complaints such as psoriasis, atopic dermatitis, contact dermatitis and seborrhoeic dermatitis.

Thus, the present invention relates to a pharmaceutical composition in the form of a gel, a cream, a lotion or a solution for topical use, comprising, in a pharmaceutically acceptable medium:
a) calcitriol, and
b) clobetasol propionate.

The calcitriol and the clobetasol propionate are present in the composition according to the invention in an amount such that they act synergistically to give the composition a therapeutic effect higher than the theoretical effect obtained by adding together the effects obtained by each of the two active agents taken separately.

The Applicant has demonstrated, surprisingly, that when thus combined in the same composition, calcitriol and clobetasol propionate are more effective in the treatment of dermatological complaints than if they are used separately.

The synergistic effect between these two active principles was observed in a model of delayed hypersensitivity reaction in mice, which constitutes an immunological response of Th1 type. This test represents a reliable model for predicting the immuno-modulatory properties of a product in a pathology of Th1 type such as psoriasis.

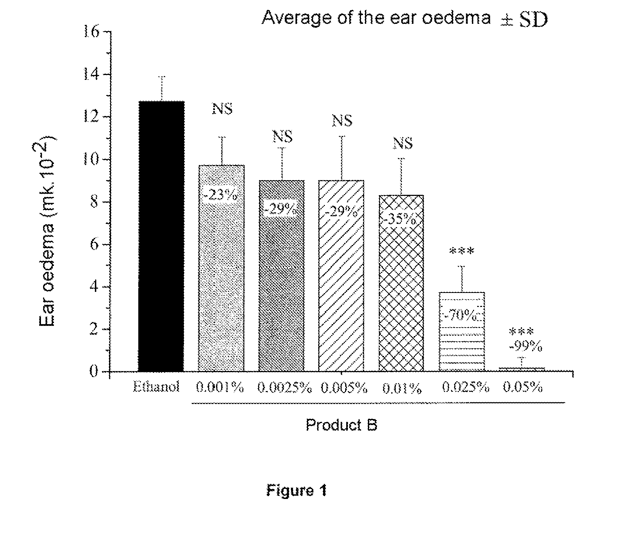

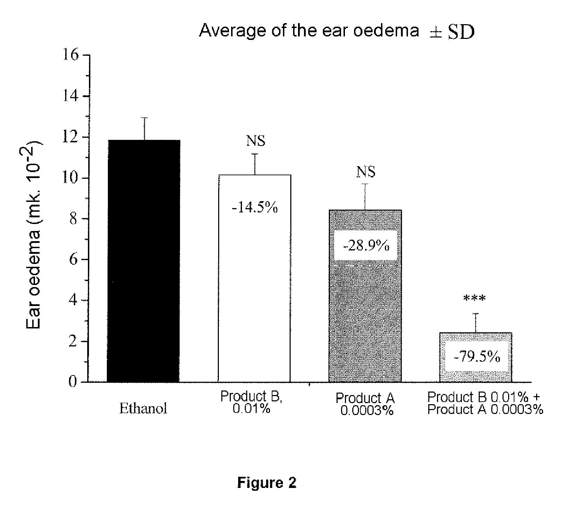

Thus, low doses of clobetasol propionate are sufficient to have an effective action when it is used in combination with calcitriol, for example, the use of 0.01% of clobetasol propionate results in a reduction of inflammation when it is combined with 0.0003% of calcitriol (Figure 2), whereas clobetasol propionate alone at this concentration shows moderate efficacy (Figures 1 and 2).

In the compositions according to the invention defined above, the calcitriol may be used at concentrations of between 0.0001 and 1 mg/g of composition.

In one preferred form of the invention, the compositions are gels, creams, lotions or solutions and contain clobetasol propionate at concentrations of between 0.01% and 2% by weight relative to the total weight of the composition.

Another subject of the invention relates to a pharmaceutical composition containing calcitriol, advantageously at concentrations of between 0.001 to 1 mg/g of composition, and 0.01% of clobetasol propionate by weight relative to the total weight of the composition.

Preferably, the composition as previously described is an ointment, a cream, a lotion a solution or a gel, advantageously an ointment.

The compositions according to the present invention are thus formulated either in the form of creams, lotions, solutions or gels, or in the form of ointments by using a suitable vehicle.

Preferably, the creams may be formed from a mixture of mineral oil, or a mixture of beeswax and water that emulsifies instantaneously, to which is added the calcitriol, dissolved in a small amount of oil such as almond oil.

Preferably, the lotions may be prepared by dissolving the calcitriol and the clobetasol propionate in an alcohol of high molecular mass, such as polyethylene glycol.

The ointments may be formulated by mixing together a solution of calcitriol and of clobetasol propionate in an oil such as almond oil, in heated paraffin, followed by allowing the mixture to cool.

The gels may preferably be prepared by dispersing or dissolving the calcitriol and the clobetasol propionate in a suitable ratio, in a gel of carbomer, poloxamer or cellulosic type.

Other ingredients may be added to the topical composition, such as preserving agents, for example DL-α-tocopherol, or fragrances, if necessary.

The present invention also relates to one of the pharmaceutical compositions as defined above, characterized in that it shows a synergistic effect between calcitriol and clobetasol propionate in the treatment of dermatological complaints such as psoriasis, atopic dermatitis, contact dermatitis and seborrhoeic dermatitis.

The present invention also relates to the use of one of the compositions as defined above for the manufacture of a medicinal product for treating dermatological complaints such as psoriasis, atopic dermatitis, contact dermatitis and seborrhoeic dermatitis, advantageously psoriasis.

The invention also relates to the use of a pharmaceutical composition in the form of an ointment for topical use comprising, in a pharmaceutically acceptable medium:
a) calcitriol, and
b) clobetasol propionate
for the manufacture of a medicinal product for treating atopic dermatitis, contact dermatitis and seborrhoeic dermatitis.

The compositions of the invention offer the following advantages over the prior art, in the case of treatment of skin complaints:
- a composition containing a combination of calcitriol and clobetasol propionate will have the advantage of having better efficacy than the use of calcitriol alone,
- the use of a combination of clobetasol propionate and calcitriol makes it possible to shorten the treatment period,
- a composition containing a mixture of calcitriol and topical clobetasol propionate, such as clobetasol propionate, makes it possible to use a lower dose of calcitriol, and thus to reduce the side effects of calcitriol (irritation and hypercalcaemia) and also the risks associated with the use of corticosteroids, in particular immune deficiency and functional modifications of the HPA axis (hypothalamo-pituitary-adrenal axis),
- the use of a combination of calcitriol and clobetasol propionate reduces the side effects of irritation of calcitriol on sensitive skin such as skin suffering from psoriasis, thus increasing the tolerance of the calcitriol treatment.

### Description of the figures

Figure 1: Dose-response effect of the topical application of clobetasol propionate (product B) on the intensity of mouse ear oedema.
   The results expressed are the average of seven mice (± SD (standard deviation)). The statistical value was determined using Student's t test (NS: non-significant p > 0.05; ***p < 0.001).
Figure 2: Synergistic effect produced by the application of a combination of calcitriol (product A) and clobetasol propionate (product B) on mouse ear oedema.
   The results expressed are the average of seven mice (± SD (standard deviation)). The statistical value was determined using Student's t test (NS: non-significant p > 0.05; ***p < 0.001).
Figure 3: Dose-response effect of the topical application of clobetasol propionate (product B) on the count of auricular ganglionic cells.
Figure 4: Synergistic effect produced by the application of a combination of calcitriol (product A) and clobetasol propionate (product B) on the count of ganglionic cells.

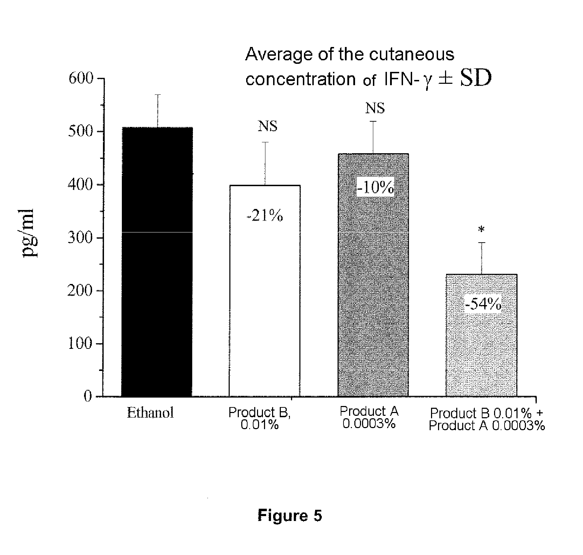

Figure 5: Synergistic effect produced by the application of a combination of calcitriol (product A) and clobetasol propionate (product B) on the cutaneous concentration of IFN-γ.

The concentrations are expressed in pg/ml ± SD. The statistical significance was determined using Student's *t* test (NS: non-significant p > 0.05; *p < 0.005).

The examples that follow illustrate, without limitation, the subject of the invention.

### Example 1: Model of mice sensitized with a hapten

For the purpose of simplicity, in the examples that follow, calcitriol is denoted as product A and clobetasol propionate as product B.

8-week-old Balb/c mice are pretreated on the abdominal skin from day 1 to day 6 using product A or B, or A and B diluted in ethanol. On day 6, the mice are actively sensitized by the topical application of 50 mg of oxazolone (oxa) in ethanol onto the abdominal skin. On day 11, an application of 20 mg of oxa in ethanol is performed on the right ear.

Student's t test was used for the statistical analysis of the results.

### 1.A. Ear oedema:

The thickness of the ear is measured, using a micrometer, on day 11 (just before the application of oxazolone to the presensitized mice) and after 24 hours, on day 12. The ear oedema is expressed as the difference between the measurement of the thickness of the ear between day 12 and day 11. The values of the thickness of the ear are analysed statistically using Student's t test. The experimental results show a dose-response effect for product (Figure 1). The 0.01% dose was chosen to perform the treatment using the combination of product A and product B, in order to be able to observe a synergistic effect. Figure 2 shows a synergistic effect during the use of a combination of product A with product B.

### 1.B. Count of the ganglionic cells:

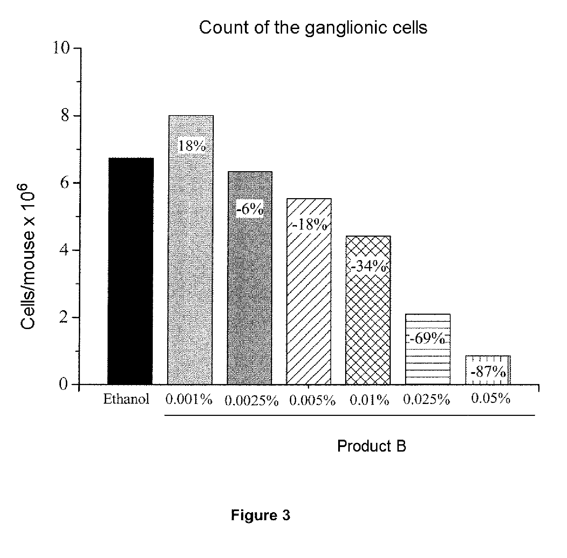

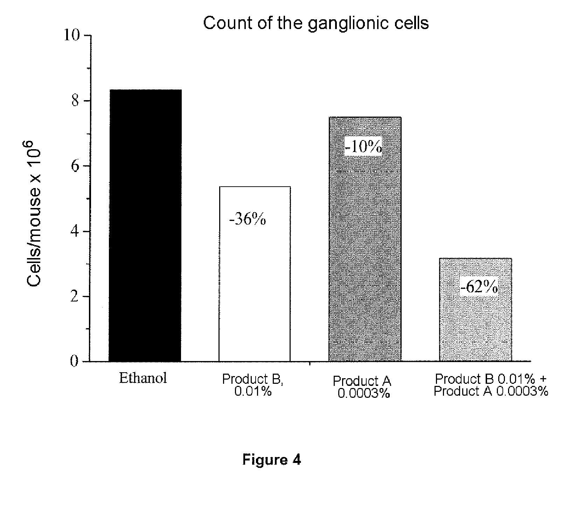

On day 12, one day after the repeated application of the oxa, the animals were sacrificed by cervical dislocation. The ganglionic cells are collected and combined by experimental group. A cell suspension is prepared and the cells are then counted. The number of cells is expressed per animal. Figure 3 shows a dose-response effect for product B. The 0.01% dose was chosen to perform the treatment using the combination of product A and product B, in order to be able to observe a synergistic effect. Figure 4 shows a synergistic effect during the use of a combination of product A with product B.

### 1.C. Determination of the cutaneous concentration of interferon-γ:

An 8 mm biopsy was performed on day 12 on the ear which received the oxazolone. After homogenization, the IFN-γ content was measured via a standard ELISA test. The results are expressed in pg/ml of homogenizate and the statistical analysis of the results is performed using Student's t test. Interferon-γ is a cytokine of Th1 type, which is strongly expressed in this animal model. The results of the experiments show a synergistic effect during the combination of product A and of product B.

## Claims

1. Pharmaceutical composition, in the form of a gel, a cream, a lotion or a solution, for topical use, comprising, in a pharmaceutically acceptable medium:
a) calcitriol, and
b) clobetasol propionate.

2. Pharmaceutical composition according to Claim 1, **characterized in that** the calcitriol is used at a concentration of between 0.001 and 1 mg/g of composition.

3. Pharmaceutical composition according to either of Claims 1 and 2, **characterized in that** the clobetasol propionate is used at a concentration of between 0.01% and 2% by weight relative to the total weight of the composition.

4. Use of a composition according to anyone of Claims 1 to 3, for the manufacture of a medicinal product for treating dermatological complaints.

5. Use of a composition according to anyone of Claims 1 to 4, for the manufacture of a medicinal product for treating atopic dermatitis, contact dermatitis and seborrhoeic dermatitis.

6. Use of a composition according to anyone of Claims 1 to 4, for the manufacture of a medicinal product for treating psoriasis.

7. Use of a pharmaceutical composition in the form of an ointment for topical use, comprising, in a pharmaceutically acceptable medium:
a) calcitriol, and
b) clobetasol propionate,
for the manufacture of a medicinal product for treating atopic dermatitis, contact dermatitis and seborrhoeic dermatitis.
